# EUROPÄISCHE PATENTSCHRIFT

(11) **EP 2 899 189 B1**
(45) Veröffentlichungstag und Bekanntmachung des Hinweises auf die Patenterteilung: **10.05.2017**
(21) Anmeldenummer: 15152138.2
(22) Anmeldetag: 22.01.2015
(51) Int. Cl.: C07D 333/22, C07D 213/48, C07D 307/46, C07F 7/08, C07C 45/49, C07C 47/21, C07C 47/24, C07C 47/232, C07C 47/277, C07C 47/238, C07C 201/12, C07C 221/00, C07C 253/30

(54) **Verfahren zur katalytischen Herstellung ungesättigter Aldehyde**
Method for the catalytic production of unsaturated aldehydes
Procédé de fabrication catalytique d'aldéhydes insaturés

(30) Priorität: 22.01.2014 DE 102014201122
(43) Veröffentlichungstag der Anmeldung: 29.07.2015
(73) Patentinhaber: Evonik Degussa GmbH, 45128 Essen (DE)
(72) Erfinder: Beller, Matthias, 18211 Nienhagen (DE); Jackstell, Ralf, 27478 Cuxhaven (DE); Fang, X., Changning District Shanghai (CN)

(56) Entgegenhaltungen:
- WO-A1-80/01691
- KR-A- 20070 017 908
- US-A- 4 687 866

## Beschreibung

Die Erfindung betrifft ein Verfahren zur katalytischen Herstellung von ungesättigten Aldehyden durch Umsetzung eines Alkens (Olefins) in Gegenwart von Synthesegas (Kohlenmonoxid und Wasserstoff), einer Rhodiumverbindung und organischen phosphorhaltigen Liganden in einem organischen Lösungsmittel sowie einem Cokatalysator aus einer schwachen organischen Säure und einem organischen Amin.

α,ß ungesättigte Aldehyde stellen eine wichtige Klasse von Feinchemikalien dar, die ein breites Anwendungsspektrum in der Nahrungsmittelindustrie, der kosmetischen und pharmazeutischen Industrie besitzen. Aufgrund ihrer vielfältigen Einsatzmöglichkeiten sind sie deshalb begehrte Zwischen- und Endprodukte in der Fein- und Großchemie. Aufgrund ihrer immensen Bedeutung sind zahlreiche Wege zu ihrer Herstellung vorgeschlagen worden. Diese schließen die Oxidation von Allylalkoholen, Petersen Olefinationen, Formylierung, Heck Reaktionen, Aldolkondensationen, Saegusa Oxidationen und Hydroformylierung von Alkinen ein. Jedes dieser Protokolle hat seine eigenen speziellen Merkmale. Einige von ihnen leiden unter gravierenden Nachteilen, wie schlechte Ausbeuten, harsche Reaktionsbedingungen, hohe Katalysatorladungen sowie Generierung von umweltschädlichen halogenierten Abfallprodukten, begrenzte Chemo-, Regio- und Stereoselektivität und aufwändige Produktisolierungsverfahren. Häufig sind auch die Ausgangstoffe schwer zugänglich. Hydroformylierung von Olefinen zu Aldehyden ist seit vielen Jahren bekannt. Aufgrund der vielfältigen Chemie der Aldehydgruppe, die weiter via Reduktion, Oxidation, oder anderen Reaktionen zu Alkoholen, Aminen, Säurederivativen, Aldolkondensationsprodukten und vielen anderen Produkten umgesetzt werden kann, sind die Aldehyde von großer Bedeutung. Die Hydroformylierung kann auch in Tandem- oder Dominoreaktionen integriert sein. Reduktion, nukleophile Addition, oder Aldolkondensation kann direkt unter den Reaktionsbedingungen der Hydroformylierung erfolgen ((a) Eilbracht, P.; Bärfacker, L.; Buss, C.; Hollmann, C.; Kitsos-Rzychon, B. E.; Kranemann, C. L.; Rische, T.; Roggenbuck, R.; Schmidt, A. Chem. Rev. 1999, 99, 3329; (b) Eilbracht, P.; Schmidt, A. M.Top. Organomet. Chem. 2006, 18, 65-95). Zusätzliche Reagentien, Produkte oder Variationen der Reaktionsbedingungen optimiert für die Hydroformylierung können den initialen Hydroformylierungsschritt, welcher nicht trivial ist, unterdrücken oder behindern. Aldolprodukte werden gewöhnlich als Nebenprodukte in Olefinumsetzungen unter Hydroformylierungsbedingungen beobachtet. Entweder kann eine Aldoladdition des enolisierten Oxoaldehydmoleküls zu einem Oxoaldehyd als Homo-Aldoladdition eintreten oder einer der beiden Reaktionspartner, die unter Hydroformylierungsbedingungen gegenwärtig sind, kann eine Cross-Aldoladdition mit den üblichen Selektivitätsproblemen eingehen.

Die Hydroformylierung/Aldolreaktion führt gewöhnlich zu einer geringen Chemoselektivität und/oder zu einer geringen Ausbeute an gewünschten ungesättigten Aldehyden, insbesondere weil die korrespondierenden gesättigten Aldehyde und Alkohole unter harschen Bedingungen stark unterdrückt sind z. B. Knifton, J. F.; Lin, J. J. J. Mol. Catal. 1993, 81, 1. Andererseits haben während der letzten zwei Jahrzehnte, Eilbracht und Mitarbeiter von intramolekularen Hydroformylierung/Aldolreaktionen zur Synthese von carbocyclischen Ringprodukten berichtet, wobei ungesättigte Silylenolether als Substrate zur Vermeidung der Chemo- und Regioselektivitätsprobleme verwendet wurden z.B. Hollmann, C.; Eilbracht, P. Tetrahedron 2000, 56, 1685. Weiterhin verwendete diese Gruppe die Strategie Metallkatalyse und Organokatalyse zu kombinieren um intermolekulare Hydroformylierung/Aldolreaktionen zu realisieren, wobei zyklische Olefine oder Styrene als Substrate zur Vermeidung der Regioselektivitätprobleme der Hydroformylierungsreaktionen und Aceton als C-Nucleophile verwendet wurden z.B. Chercheja, S.; Rothenbücher, T.; Elibracht. P.*Adv. Synth. Catal.*2009*, 351*, 339.

In US 4,687,866 und WO 80/01691 wird ein Verfahren zur Herstellung von Aldehyden beschrieben. Die Reaktionsbedingungen sind hierbei so gewählt, dass das entstehende Produkt zumindest teilweise durch Verdampfen aus dem Reaktionsgemisch entfernt wird.

Jedoch war keine selektive und effiziente intermolekulare Hydroformylierung/Aldolreaktion von Olefinen zu *α,β*-ungesättigten Aldehyden ausführbar und stellt bis heute ein schwieriges Problem dar.

Die Aufgabe der Erfindung bestand deshalb darin, ungesättigte Aldehyde durch eine einfache Reaktion aus gut zugänglichen Ausgangsverbindungen zu synthetisieren.

Es wurde gefunden, dass man ungesättigte α,ß*-*Aldehyde in einem Verfahrensschritt bei hohen Selektivitäten, Umsätzen und Standzeiten erhält, wenn man ein Alken (Olefin) in Gegenwart eines Synthesegases aus Kohlenmonoxid und Wasserstoff und eines Rhodiumkatalysators in Kombination mit einem organischen Phosphorliganden in einem organischen Lösungsmittel sowie mit einem Cokatalysator aus einem organischen Amin und einer schwachen organischen Säure umsetzt. Die Reaktion verläuft über Hydroformylierung des Olefins zum Aldehyd gefolgt von Aldolkondensation zu den Zielverbindungen.

Olefine, die erfindungsgemäß als Ausgangsstoffe umgesetzt werden, können terminale Alkene, Cycloalkene und aromatische und heteroaromatische Olefine mit einer Kohlenstoffzahl von 2 bis 40 oder Gemische davon sein, die verzweigt und/oder substituiert sein können. Alken und Olefin werden als synonyme Begriffe verwendet.

Diese Substituenten können ausgewählt sein aus der Gruppen umfassend Alkylgruppen mit bevorzugt 1 bis 12 C-Atomen, Cycloalkylgruppen mit bevorzgut 4 bis 10 C-Atomen, Alkenylgruppen mit bevorzugt 1 bis 12 C-Atomen, Hydroxygruppe, Ethergruppen, Estergruppen, Carboxylgruppen, Nitrogruppen, Aminogruppen, Halogene (Cl, F, Br, J), Aryl,-Aralkylgruppen sowie heteroaromatische Kohlenwasserstoffe und Alkylreste mit bevorzugt C1-C6 Atomen, die ihrerseits mit den genannten Substituenten substituiert sein können. Aryl bedeutet z.B. Phenyl, Naphthyl, Anthryl. Heteroaromatische Verbindungen sind Heteroarylreste, deren Ringgerüst ein oder mehrere Heteroatome (O, N, S) enthält.

Das organische Lösungsmittel ist vorzugsweise ausgewählt aus der Gruppe umfassend Essigsäure(methyl, ethyl oder n-butyl)ester und N-Methylpyrrolidon. Besonders bevorzugt ist Essigsäureethylester.

Der Rhodiumkatalysator fungiert als Hydroformylierungskatalysator. Bevorzugt ist ein Rhodiumkomplex, der mindestens eine der folgenden Verbindungen enthält: CO und/oder Olefin, z.B. Cyclooctadienyl, Norbornadienyl, Ethenyl, Cyclopentadienyl, Halogenid, z. B. Cl⁻, Br⁻, Tetrafluoroborat, Hydrid (H⁻), Carboxylat, z. B. Acetat, Acetylacetonat, Nonanoat und/oder Sulfat. Besonders bevorzugt eingesetzte Rhodiumkomplexe sind z.B. Rh(CO)₂(acac), Rh(II)acetat (dimer), RhCODBF₄, RhNBD BF₄, Rh (II)octanoat (dimer), Hexarhodium-hexadecacarbonyl. Ganz besonders bevorzugt wird z.B. Rh(CO)₂(acac) eingesetzt.

Als Liganden können beliebige Phosphor-enthaltende Liganden eingesetzt werden, die eine koordinative Bindung zum Rhodiumzentrum ausbilden können. Die Liganden können dabei sowohl monodentat als auch multidentat binden. Bevorzugt handelt es sich um einzähnige oder zweizähnige Phosphor-haltige Liganden, so z.B. monodentate Phosphine, Phosphite, Phosphonite, Phosphinite oder bidentate Phosphine, Phosphonite, Phospite, Phosphinite bzw. gemischte bidentate Liganden wie Phosphin/Phosphitkombinationen, wobei der Phosphor an Aryl- und/oder (Cyclo)alkyl-, Aryloxy- und/oder (Cyclo)alkoxygruppen gebunden ist.

Besonders bevorzugte Liganden sind ausgewählt aus der Gruppe L1 bis L7:

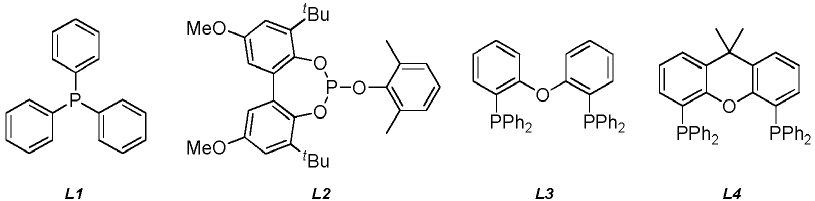

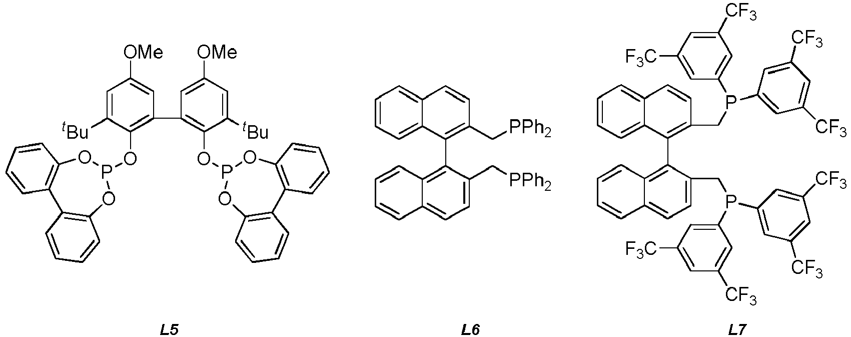

Besonders effektiv ist NAPHOS (**L6** = 2,2'-Bis(diphenylphosphino)methyl-1,1'-binaphthyl) als bidentater Diphosphinligand im erfindungsgemäßen Verfahren einsetzbar.

Das Verhältnis von Rhodium : Ligand sollte bei der Verwendung monodentater Liganden vorzugsweise 1: 50, bevorzugt 1:10, und bei der Verwendung bidentater Liganden 1:10, bevorzugt 1:2 betragen.

Der verwendete Cokatalysator stellt eine Kombination aus einem organischen Amin und einer schwachen organischen Säure dar, vorzugsweise in einem Molverhältnis von 2:1 bis 1:2, besonderes bevorzugt im Verhältnis 1:1.

Als organische Amine werden vorzugsweise primäre oder sekundäre Amine verwendet. Besonderes bevorzugt sind die Amine ausgewählt aus der Gruppe umfassend Pyrrolidin, Piperidin, C₁-C₁₀-Alkyl(oder C₅-C₁₀-cycloalkyl)- oder C₁-C₁₀- Dialkyl (oder C₅-C₁₀-cycloalkyl)amin und Morpholin sowie Piperazin, wobei die Verbindungen substituiert sein können. Besonders bevorzugt ist Pyrrolidin.

Die schwache organische Säure ist bevorzugt ausgewählt aus der Gruppe umfassend C₆-C₁₅-aromatische Carbonsäuren, aliphatische C₁-C₂₀-Carbonsäuren, und heteroaromatische Carbonsäuren, wobei die Verbindungen substituiert sein können. Aromatische (Kohlenwasserstoff-)Reste sind Arylreste, z. B. Phenyl, Naphthyl, Anthryl.

Bei heteroaromatischen Verbindungen handelt es sich um aromatische Verbindungen, deren Ringgerüst ein oder mehrere Heteroatome (O, N, S) enthält.

Aromatische Carbonsäuren sind z.B. Benzoesäuren und Naphthoesäure. Als aliphatische Carbonsäuren seien z. B. Essigsäure, Nonansäure (Pelargonsäure), Adipinsäure erwähnt. Heteroaromatische Carbonsäuren sind z. B. Thiophencarbonsäure, Nicotinsäure, Furan-2-carbonsäure. Besonders bevorzugt sind Benzoesäuren, Essigsäure, Nonansäure und Thiophensäure.

Als Substituenten kommen die bereits genannten anderen (C₁ bis Z₁₂) Alkyl- oder (C₄ bis C₈) Cycloalkylgruppen, (C₁ bis C₁₂) Alkenylgruppen, Ethergruppen, Estergruppen, Hydroxygruppe, Carboxylgruppen, Nitrogruppen, Aminogruppen, Halogene (F, Cl, Br, J), aromatische Kohlenwasserstoffe (Aryl- und Aralkylgruppen) und heteroaromatische Kohlenwasserstoffe in Frage.

Besonderes bevorzugt wird im erfindungsgemäßen Verfahren als Cokatalysator die Kombination Pyrrolidin/Benzoesäure, Pyrrolidin/4-Methoxybenzoesäure, Pyrrolidin/4-(Trifluoromethyl)benzoesäure, Pyrrolidin/Essigsäure, Pyrrolidin/Nonansäure oder Pyrrolidin/3-Thiophencarbonsäure eingesetzt. Ganz besonders bevorzugt ist die Kombination Pyrrolidin/Benzoesäure.

Überraschend führt weder der Einsatz einer starken Base wie NaOH oder eines organischen Amins allein noch der Einsatz einer schwachen organischen Säure allein zu den gewünschten Zielverbindungen. Auch bei Abwesenheit eines Phosphorliganden findet die erfindungsgemäße Reaktion zu gewünschten ungesättigten Aldehyden nicht statt.

Erst die Kombination aller Komponenten führt zu einer hochselektiven Synthese von ungesättigten Aldehyden durch Umsetzung eines Olefins in Gegenwart von Kohlenmonoxid und Wasserstoff.

Bei dem erfindungsgemäßen Verfahren liegt das Molverhältnis von Kohlenmonoxid zu Wasserstoff zweckmäßigerweise im Bereich von 10:1 bis 1:10, bevorzugt von 1:2. Am geeignetsten ist ein Verhältnis von 1:1, da in diesem Fall kein überschüssiges Kohlenmonoxid oder überschüssiger Wasserstoff aus dem Gefäß, in dem die Reaktion stattfindet, ausgeschleust werden muss.

Vorzugsweise liegt die Reaktionstemperatur bei der das Verfahren durchgeführt wird im Bereich von 20 bis 150°C, bevorzugt bei 50 bis 110°C.

Vorzugsweise liegt der Druck bei dem das Verfahren durchgeführt wird im Bereich von 1 bis 50 bar, vorzugsweise bei 3 bis 12 bar.

Das Verfahren ist effizient und selektiv und verläuft unter milden Bedingungen. Es erlaubt gute Ausbeuten an entsprechenden ungesättigten Aldehyden bei effektiver Unterdrückung von ungewünschten Homo-Aldolnebenreaktionen.

Überraschend wurde darüber hinaus gefunden, dass mit dem vorliegenden Verfahren ebenfalls Kreuzaldolkondensate hergestellt werden können, wenn ein Olefin mit einem entsprechenden aromatischen oder heteroaromatischem Aldehyd unter den gleichen Bedingungen wie für die oben beschriebene Reaktion umgesetzt wird. Das erfindungsgemäße Verfahren wird demzufolge auch zur Herstellung von Kreuzaldolkondensaten verwendet. Beliebige terminale Alkene, Cycloalkene oder aromatische Olefine bevorzugt mit einer Kohlenstoffzahl von 2 bis 40 oder Gemische davon, die gegebenenfalls substituiert sein können, werden mit einem beliebigen aromatischen oder heteroaromatischem Aldehyd, dessen Aryl- oder Heteroarylrest gegebenenfalls ebenfalls substituiert sein kann, in Gegenwart von Kohlenmonoxid und Wasserstoff sowie einem der o.g. Rhodiumkatalysatoren in Kombination mit einem der o.g. organischen Phosphorliganden und einem o.g. Cokatalysator aus einem organischen Amin und einer schwachen organischen Säure in einem polaren Lösungsmittel wie NMP (N-Methylpyrrolidon) oder DMF (Dimethylformamid) bzw. ohne Lösungsmittel umgesetzt. Als Substituenten fungieren alle bereits genannten. Besonders bevorzugt findet die Reaktion in NMP statt. Es werden entsprechende Kreuzaldolkondensate mit guten Ausbeuten erhalten.

Die nachstehenden Beispiele dienen zur Erläuterung des erfindungsgemäßen Verfahrens. Aus den Beispielen sind die sehr guten Ausbeuten und Selektivitäten für das erfindungsgemäße Verfahren deutlich erkennbar.

### Ausführungsbeispiele

### Beispiel 1

Umsetzung von 1-Octen (**1a**) unter Verwendung von Rh(CO)₂(acac) und verschiedener Liganden L1 - L7 sowie der in Tabelle 1 dargestellten Cokatalysatoren zum Zielprodukt **3a** gemäß folgender Reaktionsgleichung:

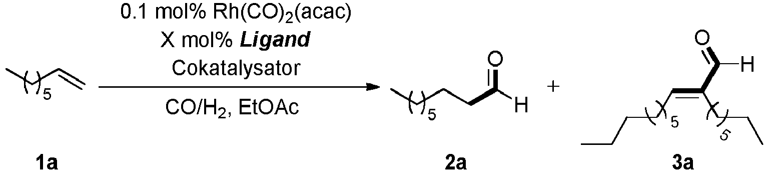

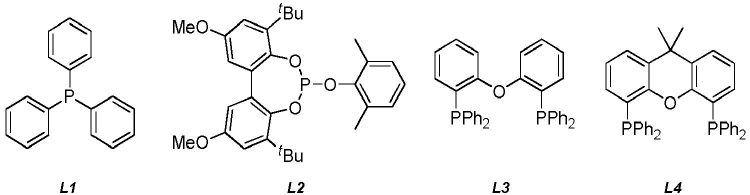

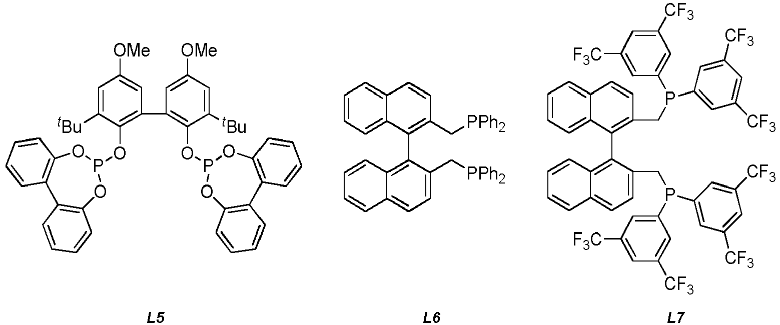

Tabelle 1 sind Cotatalysatoren, Liganden, Umsatzraten und Ausbeute zu entnehmen.

**Tabelle 1**

| Beispiel[a] | Cokatalysator | Ligand | Umsatz. | 2a Ausbeute (*n*/*iso*) | 3a Ausbeute (*E*/*Z*) |
|---|---|---|---|---|---|
| **1.1** | NaOH (10 mol%) | ***L6*** | 99% | 96% (76/24) | 0% |
| **1.2** | *L*-Prolin (10 mol%) | ***L6*** | 87% | 76% (93/7) | 3% (89/11) |
| **1.3** | Pyrrolidin (10 mol%) | ***L6*** | 97% | 79% (98/2) | 4% (99/1) |
| **1.4** | Benzoesäure (10 mol%) | ***L6*** | 98% | 95% (95/5) | 0% |
| **1.5** | Pyrrolidin (10 mol%) und Benzoesäure (10 mol%) | ***L6*** | 100% | 3% (66/34) | 90% (96/4) |
| **1.6** | Pyrrolidin (10 mol%) und Benzoesäure (10 mol%) | - | 12% | 0% | 0% |
| **1.7** | Pyrrolidin (10 mol%) und Benzoesäure (10 mol%) | ***L3*** | 36% | 10% (62/38) | 17% (>99/1) |
| **1.8** | Pyrrolidin (10 mol%) und Benzoesäure (10 mol%) | ***L4*** | 86% | 17% (50/50) | 39% (94/6) |
| **1.9** | Pyrrolidin (10 mol%) und Benzoesäure (10 mol%) | ***L5*** | 99% | 4% (1/99) | 58% (96/4) |
| **1.10** | Pyrrolidin (10 mol%) und Benzoesäure (10 mol%) | ***L7*** | 99% | 0% | 80% (96/4) |
| **1.11** | Pyrrolidin (5 mol%) und Benzoesäure (5 mol%) | ***L6*** | 98% | 3% (66/34) | 90% (96/4) |
| **1.12** | Pyrrolidin (2.5 mol%) und Benzoesäure (2.5 mol%) | ***L6*** | 100% | 14% (88/12) | 70% (95/5) |
| **1.13** | Pyrrolidin (5 mol%) und Benzoesäure (5 mol%) | ***L6*** | 98% | 0% | 68% (96/4) |

| | | | | | |
|---|---|---|---|---|---|
| [a], 65 °C, 24 h, 10 bar Synthesegas (CO:H₂ = 1: 1) | | | | | |

Beispiel 1.1 (Tabelle 1): Ein 25 ml Schlenkgefäß wird mit [Rh(CO)₂(acac)] (3,1 mg, 0,1 mol%), **L6** (15,6 mg, 0,2 mol%) und EtOAc (16 ml) unter Argon befüllt. Ein Vial (4 ml) wird mit NaOH (6 mg, 10 mol%) befüllt und ein Magnetrührer wird hinzugefügt. Anschließend werden 2 ml der gelben Lösung aus dem Schlenkgefäß und 1**a** (235 µl, 1,5 mmol) mittels einer Spritze in das Vial überführt. Dieses Vial wird in einer Edelstahlplatte platziert, welche dann unter Argon in einen 300 ml Autoklaven der Serie 4560 von Parr Instruments überführt wird. Nach dem dreimaligen Spülen des Autoklavens mit Stickstoff werden 10 bar Synthesegas (CO : H₂ = 1: 1) aufgepresst. Dann wird die Reaktion 24 Stunden bei 65 °C durchgeführt. Nach dem Ende der Reaktion wird der Autoklav auf Raumtemperatur abgekühlt und langsam entspannt. Es wird Isooctan als interner Standard hinzugefügt und die Ausbeute, der Umsatz und die Selektivität mittels GC Analyse bestimmt.
Beispiel 1.2 (Tabelle 1): Analog Beispiel 1.1, anstelle NaOH wird *L*-Prolin (17,3 mg, 10 mol%) verwendet.
Beispiel 1.3 (Tabelle 1): Analog Beispiel 1.1, anstelle NaOH wird Pyrrolidin (12,3 µL, 10 mol%) verwendet.
Beispiel 1.4 (Tabelle 1): Analog Beispiel 1.1, anstelle NaOH wird Benzoesäure (18,3 mg, 10 mol%) verwendet.
Beispiel 1.5 (Tabelle 1): Analog Beispiel 1.1, anstelle NaOH werden Pyrrolidin (12,3 µl, 10 mol%) und Benzoesäure (18,3 mg, 10 mol%) verwendet.
Beispiel 1.6 (Tabelle 1): Ein 25 ml Schlenkgefäß wird mit [Rh(CO)₂(acac)] (3,1 mg, 0,1 mol%), Pyrrolidin (100 µl, 10 mol%), Benzoesäure (146,4 mg, 10 mol%) und EtOAc (16 ml) unter Argon befüllt. Anschließend werden 2 ml der gelben Lösung aus dem Schlenkgefäß und 1**a** (235 µl, 1.5 mmol) mittels einer Spritze in ein 4 ml Vial überführt und ein Magnetrührer hinzugefügt. Dieses Vial wird in einer Edelstahlplatte platziert, welche dann unter Argon in einen 300 ml Autoklaven der Serie 4560 von Parr Instruments überführt wird. Nach dem dreimaligen Spülen des Autoklavens mit Stickstoff werden 10 bar Synthesegas (CO : H₂ = 1: 1) aufgepresst. Dann wird die Reaktion 24 Stunden bei 65 °C durchgeführt. Nach dem Ende der Reaktion wird der Autoklav auf Raumtemperatur abgekühlt und langsam entspannt. Es wird Isooctan als interner Standard hinzugefügt und die Ausbeute, der Umsatz und die Selektivität mittels GC Analyse bestimmt.
Beispiel 1.7 (Tabelle 1): Ein 25 ml Schlenkgefäß wird mit [Rh(CO)₂(acac)] (3,1 mg, 0,1 mol%), Pyrrolidin (100 µL, 10 mol%), Benzoesäure (146,4 mg, 10 mol%) und EtOAc (16 ml) unter Argon befüllt. Ein Vial (4 ml) wird mit **L3** (1,62 mg, 0.2 mol%) befüllt und ein Magnetrührer wird hinzugefügt. Anschließend werden 2 ml der gelben Lösung aus dem Schlenkgefäß und 1a (235 µl, 1,5 mmol) mittels einer Spritze in das Vial überführt. Dieses Vial wird in einer Edelstahlplatte platziert, welche dann unter Argon in einen 300 ml Autoklaven der Serie 4560 von Parr Instruments überführt wird. Nach dem dreimaligen Spülen des Autoklavens mit Stickstoff werden 10 bar Synthesegas (CO : H₂ = 1: 1) aufgepresst. Dann wird die Reaktion 24 Stunden bei 65 °C durchgeführt. Nach dem Ende der Reaktion wird der Autoklav auf Raumtemperatur abgekühlt und langsam entspannt. Es wird Isooctan als interner Standard hinzugefügt und die Ausbeute, der Umsatz und die Selektivität mittels GC Analyse bestimmt.
Beispiel 1.8 (Tabelle 1): Analog Beispiel 1.7, anstelle L3 wird **L4** (1,74 mg, 0,2 mol%) verwendet.
Beispiel 1.9 (Tabelle 1): Analog Beispiel 1.7, anstelle L3 wird **L5** (2,36 mg, 0,2 mol%) verwendet.
Beispiel 1.10 (Tabelle 1): Analog Beispiel 1.7, anstelle L3 wird **L7** (3,58 mg, 0,2 mol%) verwendet.
Beispiel 1.11 (Tabelle 1): Ein 25 ml Schlenkgefäß wird mit [Rh(CO)₂(acac)] (3,1 mg, 0,1 mol%), Pyrrolidin (50 µl, 5 mol%), Benzoesäure (73,2 mg, 5 mol%) und EtOAc (16 ml) unter Argon befüllt. Ein Vial (4 ml) wird mit **L6** (1,95 mg, 0,2 mol%) befüllt und ein Magnetrührer wird hinzugefügt. Anschließend werden 2 ml der gelben Lösung aus dem Schlenkgefäß und 1**a** (235 µl, 1,5 mmol) mittels einer Spritze in das Vial überführt. Dieses Vial wird in einer Edelstahlplatte platziert, welche dann unter Argon in einen 300 ml Autoklaven der Serie 4560 von Parr Instruments überführt wird. Nach dem dreimaligen Spülen des Autoklavens mit Stickstoff werden 10 bar Synthesegas (CO : H₂ = 1: 1) aufgepresst. Dann wird die Reaktion 24 Stunden bei 65 °C durchgeführt. Nach dem Ende der Reaktion wird der Autoklav auf Raumtemperatur abgekühlt und langsam entspannt. Es wird Isooctan als interner Standard hinzugefügt und die Ausbeute, der Umsatz und die Selektivität mittels GC Analyse bestimmt.
Beispiel 1.12 (Tabelle 1): Analog Beispiel 1.11, es werden jedoch Pyrrolidin (25 µl, 2,5 mol%) und Benzoesäure (36,6 mg, 2,5 mol%) verwendet.
Beispiel 1.13 (Tabelle 1): ): Analog Beispiel 1.11, jedoch werden nach dem dreimaligen Spülen des Autoklavens mit Stickstoff 5 bar Synthesegas (CO : H₂ = 1: 1) aufgepresst.

### Beispiel 2

Umsetzung von 1-Octen unter Verwendung von Rh(CO)₂(acac) und L6 gemäß folgender Gleichung sowie unter Verwendung der in Tabelle 2 dargestellten Cokatalysatoren:

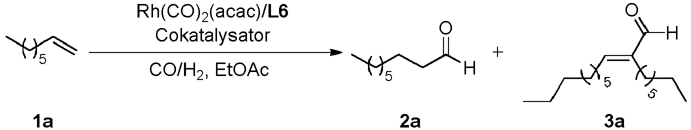

**Tabelle 2**

| Beispiel | Cokatalysator | Umsatz | 2a Ausbeute (*n*/*iso*) | 3a Ausbeute (*E*/*Z*) |
|---|---|---|---|---|
| **2.1** | Pyrrolidin und Benzoesäure | 98% | 3% (66/34) | 90% (96/4) |
| **2.2** | Piperidin und Benzoesäure | 98% | 27% (63/37) | 62% (96/4) |
| **2.3** | Diethylamin und Benzoesäure | 98% | 27% (93/7) | 68% (94/6) |
| **2.4** | *n*-Butylamin und Benzoesäure | 89% | 26% (65/35) | 40% (95/5) |
| **2.5** | Pyrrolidin und AcOH | 97% | 14% (88/12) | 81% (97/3) |
| **2.6** | Pyrrolidin und Nonansäure | 100% | 3% (65/35) | 90% (97/3) |
| **2.7** | Pyrrolidin und 4-Methoxybenzoesäure | 98% | 6% (79/21) | 89% (97/3) |
| **2.8** | Pyrrolidin und 4-(Trifluoromethyl)benzoesäure | 97% | 5% (79/21) | 89% (98/2) |
| **2.9** | Pyrrolidin und 3-Thiophencarbonsäure | 87% | 6% (75/25) | 79% (98/2) |

Beispiel 2.1 (Tabelle 2): Ein 25 ml Schlenkgefäß wird mit[Rh(CO)₂(acac)] (3,1 mg, 0,1 mol%), **L6** (15,6 mg, 0,2 mol%) und EtOAc (16 ml) unter Argon befüllt. Ein Vial (10 ml) wird mit Benzoesäure (18,3 mg, 5 mol%) befüllt und ein Magnetrührer wird hinzugefügt. Anschließend werden 4 ml der gelben Lösung aus dem Schlenkgefäß und 1**a** (471 µL, 3 mmol) und Pyrrolidin (12,3 µL, 5 mol%) mittels einer Spritze hinzugefügt. Dieses Vial wird in einer Edelstahlplatte platziert, welche dann unter Argon in einen 300 ml Autoklaven der Serie 4560 von Parr Instruments überführt wird. Nach dem dreimaligen Spülen des Autoklavens mit Stickstoff werden 10 bar Synthesegas (CO : H₂ = 1: 1) aufgepresst. Dann wird die Reaktion 24 Stunden bei 65 °C durchgeführt. Nach dem Ende der Reaktion wird der Autoklav auf Raumtemperatur abgekühlt und langsam entspannt. Es wird Isooctan als interner Standard hinzugefügt und die Ausbeute, der Umsatz und die Selektivität mittels GC Analyse bestimmt.
Beispiel 2.2 (Tabelle 2): Ein 25 ml Schlenkgefäß wird mit[Rh(CO)₂(acac)] (3,1 mg, 0,1 mol%), **L6** (15,6 mg, 0,2 mol%) und EtOAc (16 ml) unter Argon befüllt. Ein Vial (10 ml) wird mit Benzoesäure (18,3 mg, 5 mol%) befüllt und ein Magnetrührer wird hinzugefügt. Anschließend werden 4 ml der gelben Lösung aus dem Schlenkgefäß und 1a (471 µl, 3 mmol) und Piperidin (14,9 µl, 5 mol%) mittels einer Spritze hinzugefügt. Dieses Vial wird in einer Edelstahlplatte platziert, welche dann unter Argon in einen 300 ml Autoklaven der Serie 4560 von Parr Instruments überführt wird. Nach dem dreimaligen Spülen des Autoklavens mit Stickstoff werden 10 bar Synthesegas (CO : H₂ = 1: 1) aufgepresst. Dann wird die Reaktion 24 Stunden bei 65 °C durchgeführt. Nach dem Ende der Reaktion wird der Autoklav auf Raumtemperatur abgekühlt und langsam entspannt. Es wird Isooctan als interner Standard hinzugefügt und die Ausbeute, der Umsatz und die Selektivität mittels GC Analyse bestimmt.
Beispiel 2.3 (Tabelle 2): ): Analog Beispiel 2.2, es wird anstelle Piperidin Diethylamin (15,7 µl, 5 mol%) mittels einer Spritze hinzugefügt.
Beispiel 2.4 (Tabelle 2): Analog Beispiel 2.2, es wird anstelle Piperidin *n*-Butylamin (14.8 µl, 5 mol%) mittels einer Spritze hinzugefügt.
Beispiel 2.5 (Tabelle 2): Ein 25 ml Schlenkgefäß wird mit [Rh(CO)₂(acac)] (3,1 mg, 0,1 mol%), **L6** (15,6 mg, 0,2 mol%) und EtOAc (16 ml) unter Argon befüllt. Ein Vial (10 ml) wird mit Pyrrolidin (100 µl, 5 mol%) befüllt und ein Magnetrührer wird hinzugefügt. Anschließend werden 4 ml der gelben Lösung aus dem Schlenkgefäß und 1**a** (471 µl, 3 mmol) und Essigsäure (8,6 µl, 5 mol%)) mittels einer Spritze hinzugefügt. Dieses Vial wird in einer Edelstahlplatte platziert, welche dann unter Argon in einen 300 ml Autoklaven der Serie 4560 von Parr Instruments überführt wird. Nach dem dreimaligen Spülen des Autoklavens mit Stickstoff werden 10 bar Synthesegas (CO : H₂ = 1: 1) aufgepresst. Dann wird die Reaktion 24 Stunden bei 65 °C durchgeführt. Nach dem Ende der Reaktion wird der Autoklav auf Raumtemperatur abgekühlt und langsam entspannt. Es wird Isooctan als interner Standard hinzugefügt und die Ausbeute, der Umsatz und die Selektivität mittels GC Analyse bestimmt.
Beispiel 2.6 (Tabelle 2): Ein 25 ml Schlenkgefäß wird mit [Rh(CO)₂(acac)] (3,1 mg, 0,1 mol%), **L6** (15,6 mg, 0,2 mol%) und EtOAc (16 ml) unter Argon befüllt. Ein Vial (10 ml) wird mit Pyrrolidin (100 µl, 5 mol%) befüllt und ein Magnetrührer wird hinzugefügt. Anschließend werden 4 ml der gelben Lösung aus dem Schlenkgefäß und 1**a** (471 µl, 3 mmol) und Nonansäure (26,4 µl, 5 mol%) mittels einer Spritze hinzugefügt. Dieses Vial wird in einer Edelstahlplatte platziert, welche dann unter Argon in einen 300 ml Autoklaven der Serie 4560 von Parr Instruments überführt wird. Nach dem dreimaligen Spülen des Autoklavens mit Stickstoff werden 10 bar Synthesegas (CO : H₂ = 1: 1) aufgepresst. Dann wird die Reaktion 24 Stunden bei 65 °C durchgeführt. Nach dem Ende der Reaktion wird der Autoklav auf Raumtemperatur abgekühlt und langsam entspannt. Es wird Isooctan als interner Standard hinzugefügt und die Ausbeute, der Umsatz und die Selektivität mittels GC Analyse bestimmt.
Beispiel 2.7 (Tabelle 2): Analog Beispiel 2.6, anstelle Nonansäure wird 4-Methoxybenzoesäure(22,8 mg, 5 mol%) mittels einer Spritze hinzugefügt.
Beispiel 2.8 (Tabelle 2): Analog Beispiel 2.6, anstelle Nonansäure wird 4-(Trifluormethyl)benzoesäure (28,5 mg, 5 mol%) mittels einer Spritze hinzugefügt.
Beispiel 2.9 (Tabelle 2): Analog Beispiel 2.6, anstelle Nonansäure wird 3-Thiophencarbonsäure(19,2 mg, 5 mol%) mittels einer Spritze hinzugefügt.

### Beispiel 3

Umsetzung verschiedener Olefine gemäß folgender Reaktionsgleichung:

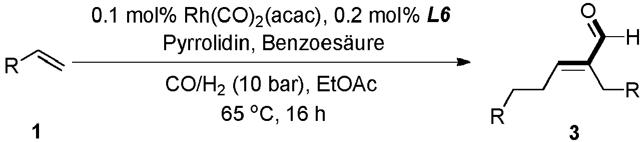

Tabelle 3 zeigt Ausgangsolefine, Produkte und Ausbeute:

**Tabelle 3**

| Beispiel | 1 | 3 | Ausbeute (*E*/*Z*) |
|---|---|---|---|
| **3.1** | 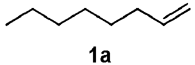 | 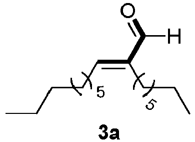 | 89% (96/4) |
| **3.2** | 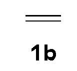 | 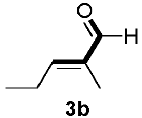 | 98% (>99/1) |
| **3.3** | 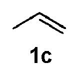 | 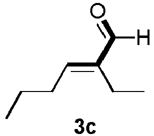 | 69% (95/5) |
| **3.4** | 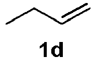 | 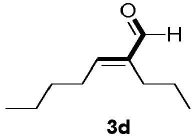 | 81% (97/3) |
| **3.5** | 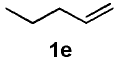 | 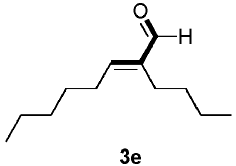 | 85% (>99/1) |
| **3.6** | 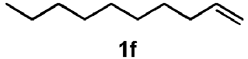 | 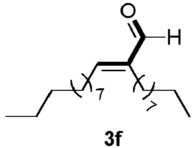 | 91% (97/3) |
| **3.7** | 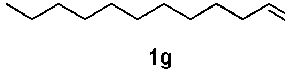 | 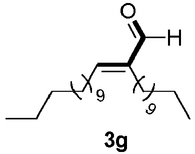 | 89% (97/3) |
| **3.8** | 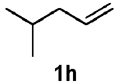 | 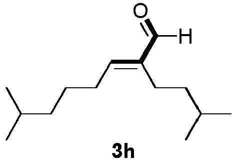 | 86% (99/1) |
| **3.9** | 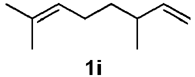 | 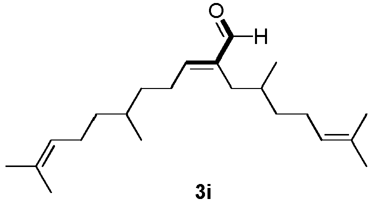 | 75% (97/3) |
| **3.10** | 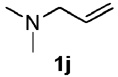 | 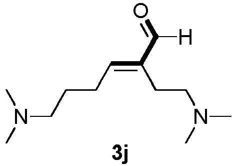 | 78% (>99/1) |
| **3.11** | 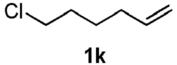 | 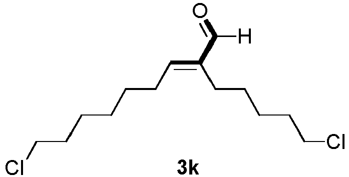 | 69% (97/3) |
| **3.12** | 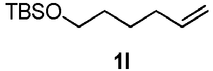 | 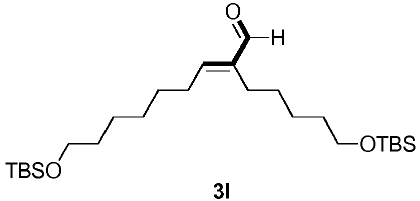 | 92% (95/5) |

| | | | |
|---|---|---|---|
| TBS = *tert*-butyldimethylsilyl | | | |

Beispiel 3.1 (Tabelle3): Ein 100 ml Stahlautoklav wird unter Argon mit [Rh(CO)₂(acac)] (3,87 mg, 0,1 mol%), **L6** (19,52 mg, 0,2 mol%) und Benzoesäure (91,5 mg, 5 mol%) befüllt. Anschließend werden EtOAc (20 ml), Pyrrolidin (62,5 µl, 5 mol%), und **1a** (2,35 ml, 15 mmol) unter Argon hinzugefügt. Der Autoklav wird mit 10 bar Synthesegas (CO : H₂ = 1: 1) befüllt und 16 Stunden bei 65 °C gerührt. Nach der Reaktion wird der Autoklav mit Eiswasser heruntergekühlt und der Druck abgelassen Die Selektivität wurde mittels GC/MS Analyse bestimmt. Der Reaktionsansatz wurde im Vakuum eingeengt und mittels Säulenchromatographie über Kieselgel (Eluent Ethylacetat: Heptan = 1:30) aufgereinigt und ergibt das Produkt **3a.** Die Ausbeute ist die isolierte Ausbeute.
Beispiel 3.2 (Tabelle3): Analog Beispiel 3.1, anstelle 1a wird **1 b** (420,8 mg, 15 mmol) unter Argon hinzugefügt. Der Autoklav wird mit 10 bar Synthesegas (CO : H₂ = 1: 1) befüllt und 16 Stunden bei 65 °C gerührt. Nach der Reaktion wird der Autoklav mit Eiswasser heruntergekühlt und der Druck abgelassen. Die Ausbeute und Selektivität wurde mittels GC/MS Analyse bestimmt.
Beispiel 3.3 (Tabelle 3): Analog Beispiel 3.1, anstelle 1a wird **1c** (631,2 mg, 15 mmol) unter Argon hinzugefügt. Der Autoklav wird mit 10 bar Synthesegas (CO : H₂ = 1: 1) befüllt und 16 Stunden bei 65 °C gerührt. Nach der Reaktion wird der Autoklav mit Eiswasser heruntergekühlt und der Druck abgelassen. Die Ausbeute und Selektivität wurde mittels GC/MS Analyse bestimmt.
Beispiel 3.4 (Tabelle 3): Analog Beispiel 3.1, anstelle 1a wird **1d** (841.7 mg, 15 mmol) unter Argon hinzugefügt. Der Autoklav wird mit 10 bar Synthesegas (CO : H₂ = 1: 1) befüllt und 16 Stunden bei 65 °C gerührt. Nach der Reaktion wird der Autoklav mit Eiswasser heruntergekühlt und der Druck abgelassen. Die Ausbeute und Selektivität wurde mittels GC/MS Analyse bestimmt. Der Reaktionsansatz wurde im Vakuum eingeengt und mittels Säulenchromatographie über Kieselgel (Eluent Ethylacetat: Heptan = 1:30) aufgereinigt und ergibt das Produkt **3d.** Die Ausbeute ist die isolierte Ausbeute.
Beispiel 3.5 (Tabelle 3): Analog Beispiel 3.1, anstelle 1a wird **1e** (1.64 ml, 15 mmol) unter Argon hinzugefügt. Der Autoklav wird mit 10 bar Synthesegas (CO : H₂ = 1: 1) befüllt und 16 Stunden bei 65 °C gerührt. Nach der Reaktion wird der Autoklav mit Eiswasser heruntergekühlt und der Druck abgelassen. Die Selektivität wurde mittels GC/MS Analyse bestimmt. Der Reaktionsansatz wurde im Vakuum eingeengt und mittels Säulenchromatographie über Kieselgel (Eluent Ethylacetat: Heptan = 1:30) aufgereinigt und ergibt das Produkt **3e.** Die Ausbeute ist die isolierte Ausbeute.
Beispiel 3.6 (Tabelle 3): Analog Beispiel 3.1, anstelle 1a wird **1f** (2,84 ml, 15 mmol) unter Argon hinzugefügt. Der Autoklav wird mit 10 bar Synthesegas (CO : H₂ = 1: 1) befüllt und 16 Stunden bei 65 °C gerührt. Nach der Reaktion wird der Autoklav mit Eiswasser heruntergekühlt und der Druck abgelassen Die Selektivität wurde mittels GC/MS Analyse bestimmt, Der Reaktionsansatz wurde im Vakuum eingeengt und mittels Säulenchromatographie über Kieselgel (Eluent Ethylacetat: Heptan = 1:30) aufgereinigt und ergibt das Produkt **3f.** Die Ausbeute ist die isolierte Ausbeute.
Beispiel 3.7 (Tabelle 3): Analog Beispiel 3.1, anstelle 1a wird **1g** (3,33 ml, 15 mmol) unter Argon hinzugefügt. Der Autoklav wird mit 10 bar Synthesegas (CO : H₂ = 1: 1) befüllt und 16 Stunden bei 65 °C gerührt. Der Autoklav wird mit 10 bar Synthesegas (CO : H₂ = 1: 1) befüllt und 16 Stunden bei 65 °C gerührt. Nach der Reaktion wird der Autoklav mit Eiswasser heruntergekühlt und der Druck abgelassen Die Selektivität wurde mittels GC/MS Analyse bestimmt, Der Reaktionsansatz wurde im Vakuum eingeengt und mittels Säulenchromatographie über Kieselgel (Eluent Ethylacetat: Heptan = 1:30) aufgereinigt und ergibt das Produkt **3g**. Die Ausbeute ist die isolierte Ausbeute.
Beispiel 3.8 (Tabelle 3): Analog Beispiel 3.1, anstelle 1a wird **1h** (1,9 ml, 15 mmol) unter Argon hinzugefügt. Der Autoklav wird mit 10 bar Synthesegas (CO : H₂ = 1: 1) befüllt und 16 Stunden bei 65 °C gerührt. Nach der Reaktion wird der Autoklav mit Eiswasser heruntergekühlt und der Druck abgelassen Die Selektivität wurde mittels GC/MS Analyse bestimmt, Der Reaktionsansatz wurde im Vakuum eingeengt und mittels Säulenchromatographie über Kieselgel (Eluent Ethylacetat: Heptan = 1:30) aufgereinigt und ergibt das Produkt **3h.** Die Ausbeute ist die isolierte Ausbeute.
Beispiel 3.9 (Tabelle 3): Analog Beispiel 3.1, anstelle 1a wird **1i** (2,73 ml, 15 mmol) unter Argon hinzugefügt. Der Autoklav wird mit 10 bar Synthesegas (CO : H₂ = 1: 1) befüllt und 16 Stunden bei 65 °C gerührt. Nach der Reaktion wird der Autoklav mit Eiswasser heruntergekühlt und der Druck abgelassen Die Selektivität wurde mittels GC/MS Analyse bestimmt, Der Reaktionsansatz wurde im Vakuum eingeengt und mittels Säulenchromatographie über Kieselgel (Eluent Ethylacetat: Heptan = 1:30) aufgereinigt und ergibt das Produkt **3i.** Die Ausbeute ist die isolierte Ausbeute.
Beispiel 3.10 (Tabelle 3): Analog Beispiel 3.1, anstelle 1a wird **1j** (1,78 ml, 15 mmol) unter Argon hinzugefügt. Der Autoklav wird mit 10 bar Synthesegas (CO : H₂ = 1: 1) befüllt und 16 Stunden bei 65 °C gerührt. Nach der Reaktion wird der Autoklav mit Eiswasser heruntergekühlt und der Druck abgelassen Die Selektivität wurde mittels GC/MS Analyse bestimmt, Der Reaktionsansatz wurde im Vakuum eingeengt und mittels Säulenchromatographie über Kieselgel (Eluent Ethylacetat: Heptan = 1:30) aufgereinigt und ergibt das Produkt **3j.** Die Ausbeute ist die isolierte Ausbeute.
Beispiel 3.11 (Tabelle 3): Analog Beispiel 3.1, anstelle 1a wird **1k** (1,99 ml, 15 mmol) unter Argon hinzugefügt. Der Autoklav wird mit 10 bar Synthesegas (CO : H₂ = 1: 1) befüllt und 16 Stunden bei 65 °C gerührt. Nach der Reaktion wird der Autoklav mit Eiswasser heruntergekühlt und der Druck abgelassen Die Selektivität wurde mittels GC/MS Analyse bestimmt, Der Reaktionsansatz wurde im Vakuum eingeengt und mittels Säulenchromatographie über Kieselgel (Eluent Ethylacetat: Heptan = 1:30) aufgereinigt und ergibt das Produkt **3k.** Die Ausbeute ist die isolierte Ausbeute.
Beispiel 3.12 (Tabelle 3): Analog Beispiel 3.1, anstelle 1a wird **1l** (3,22 g, 15 mmol) unter Argon hinzugefügt. Der Autoklav wird mit 10 bar Synthesegas (CO : H₂ = 1: 1) befüllt und 16 Stunden bei 65 °C gerührt. Nach der Reaktion wird der Autoklav mit Eiswasser heruntergekühlt und der Druck abgelassen Die Selektivität wurde mittels GC/MS Analyse bestimmt, Der Reaktionsansatz wurde im Vakuum eingeengt und mittels Säulenchromatographie über Kieselgel (Eluent Ethylacetat: Heptan = 1:30) aufgereinigt und ergibt das Produkt **3l.** Die Ausbeute ist die isolierte Ausbeute.

### Beispiel 4

Umsetzung der Verbindungen 1a und 4a zur Herstellung von Kreuzaldolkondensaten gemäß folgender Reaktionsgleichung unter Verwendung von NMP als Lösungsmittel

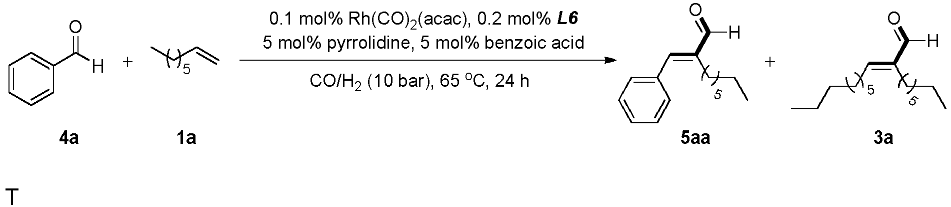

**Tabelle 4**

| Beispiel | Lösungsmittel | Umsatz | 5aa Ausbeute (*E*/*Z*) | 3a Ausbeute (E/Z) |
|---|---|---|---|---|
| 4.1 | NMP | 94% | 90% (96/4) | Trace |

| | | | | |
|---|---|---|---|---|
| NMP = N-Methyl-2-pyrrolidon | | | | |

Beispiel 4.1 (Tabelle 4): Ein Vial (4 ml) wird [Rh(CO)₂(acac)] (0,387 mg, 0.1 mol%), **L6** (1,95 mg, 0,2 mol%) und Benzoesäure (9,15 mg, 5 mol%), NMP (2 ml), **1a** (235 µl, 1.5 mmol) und **4a** (153 µl, 1.5 mmol) befüllt und ein Magnetrührer wird hinzugefügt. Dieses Vial wird in einer Edelstahlplatte platziert welche dann unter Argon in einen 300 ml Autoklaven der Serie 4560 von Parr Instruments überführt wird. Nach dem dreimaligen Spülen des Autoklavens mit Stickstoff werden 10 bar Synthesegas (CO : H₂ = 1: 1) aufgepresst. Dann wird die Reaktion 24 Stunden bei 65 °C durchgeführt. Nach dem Ende der Reaktion wird der Autoklav auf Raumtemperatur abgekühlt und langsam entspannt. Es wird Isooctan als interner Standard hinzugefügt und die Ausbeute, der Umsatz und die Selektivität mittels GC Analyse bestimmt.

### Beispiel 5

Herstellung von Kreuzaldolkondensaten aus Alkenen **1** und Aldehyden **4** gemäß Schema 1: 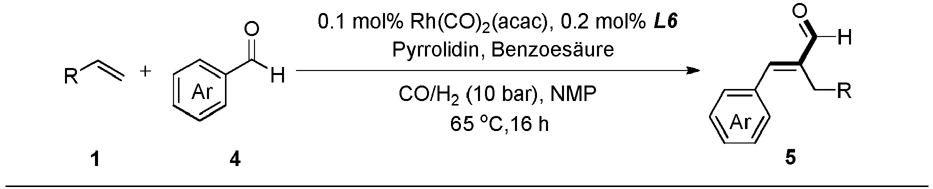 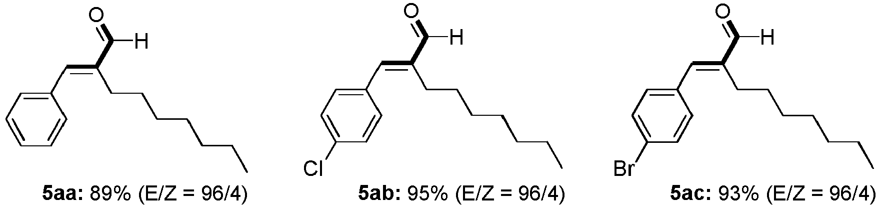 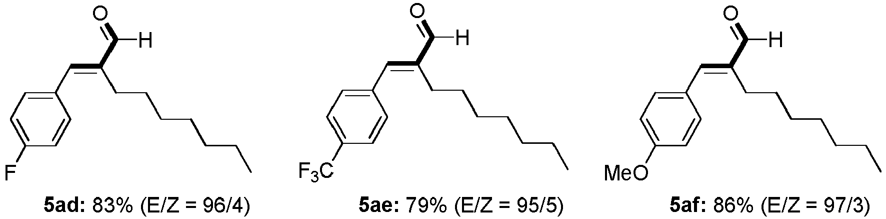 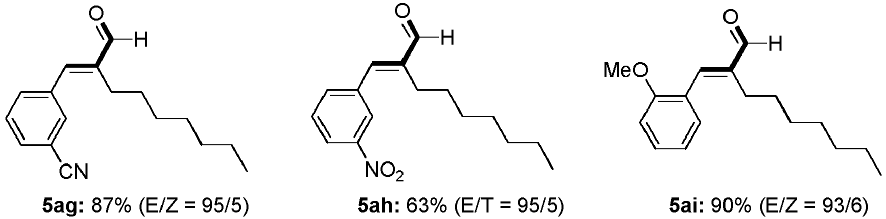 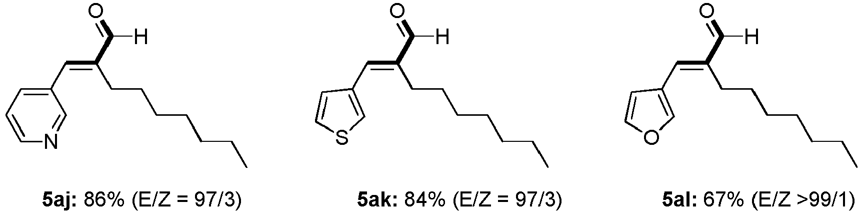 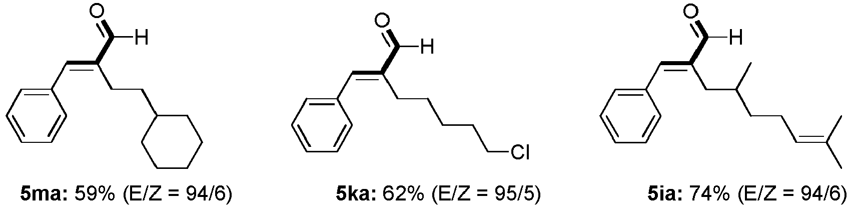
(Schema 1, **5aa**): Ein 25 ml Schlenkgefäß wird mit [Rh(CO)₂(acac)] (3,1 mg, 0,1 mol%), **L6** (15,6 mg, 0,2 mol%), Pyrrolidin (50 µl, 5 mol%), Benzoesäure (73,2 mg, 5 mol%) und NMP (16 ml) unter Argon befüllt. Ein Magnetrührer wird in ein 4 ml Vial gegeben und 2 ml der gelben Lösung aus dem Schlenkgefäß sowie 1a = 1 = 1-Octen mit R= C₆H₁₃ (235 µl, 1,5 mmol) und 4a = 4 = Benzaldehyd (153 µl, 1,5 mmol) werden mittels einer Spritze in das Vial überführt. Das Vial wird in einer Stahlplatte platziert welche in einen 300 ml Parr Autoklaven der 4560 Serie von Parr Instruments unter Argon überführt wird. Nach dreimaligem Spülen des Autoklaven mit Stickstoff, werden 10 bar Synthesegas (CO:H₂ = 1:1) aufgepresst. Die Reaktion wird 16 Stunden bei 65 °C durchgeführt, anschließend wird der Autoklav auf Raumtemperatur heruntergekühlt und der Druck vorsichtig abgelassen. Die Stereoselektivität wurde mittels GC/MS Analyse bestimmt. Die Reaktionslösung wird mit 2 ml Wasser versetzt und dreimal mit jeweils 15 ml Ethylacetat extrahiert. Die vereinigten organischen Phasen werden mit 45 ml gesättigter Kochsalzlösung gewaschen und über Magnesiumsulfat getrocknet. Nach dem Entfernen des Lösungsmittels wird der Rückstand mittels Säulenchromatographie über Kieselgel (Eluent, Ethylacetat : Heptan = 1 : 30) aufgereinigt und ergibt das Produkt **5aa.**
(Schema 1, **5ab**): Analog 5aa anstelle Benzaldehyd wird p-Chlorbenzaldehyd = **4b** (210,9 mg, 1,5 mmol) verwendet und ergibt Produkt **5ab**.
(Schema 1, **5ac**): Analog 5aa anstelle Benzaldehyd wird p-Brombenzaldehyd = **4c** (277,5 mg, 1,5 mmol) und ergibt das Produkt **5ac**.
(Schema 1, **5ad)**: Analog 5aa anstelle Benzaldehyd wird p-Fluorbenzaldehyd = **4d** (186.2 mg, 1.5 mmol) verwendet und ergibt das Produkt **5ad**.
(Schema 1, **5ae)**: Analog 5aa anstelle Benzaldehyd wird p-Trifluormethylbenzaldehyd = **4e** (261,2 mg, 1,5 mmol) verwendet und ergibt das Produkt **5ae**.
(Schema 1, **5af**): Analog 5aa anstelle Benzaldehyd wird p-Methoxybenzaldehyd **4f** (204,2 mg, 1,5 mmol) verwendet und ergibt das Produkt **5af.**
(Schema 1, **5ag**): Analog 5aa anstelle Benzaldehyd wird m-Cyanobenzaldehyd = **4g** (196,7 mg, 1,5 mmol) verwendet und ergibt das Produkt **5ag**.
(Schema 1, **5ah**): Analog 5aa anstelle Benzaldehyd wird m-Nitrobenzaldehyd = **4h** (226,7 mg, 1,5 mmol) verwendet und ergibt das Produkt **5ah**.
(Schema 1, **5ai**): Analog 5aa anstelle Benzaldehyd wird o-Methoxybenzaldehyd = **4i** (204,2 mg, 1,5 mmol) verwendet und ergibt das Produkt **5ai**.
(Schema 1, **5ai**): Analog 5aa anstelle Benzaldehyd wird Pyridin-3-carbaldehyd = **4j** (160,7 mg, 1,5 mmol) verwendet und ergibt das Produkt **5aj**.
(Schema 1, **5ak)**: Analog 5aa anstelle Benzaldehyd wird Thiophen-3-aldehyd = **4k** (168,2 mg, 1,5 mmol) verwendet und ergibt das Produkt **5ak**.
(Schema 1, **5al**): Analog 5aa anstelle Benzaldehyd wird Furan-3-aldehyd = **4l** (144.1 mg, 1.5 mmol) verwendet und ergibt das Produkt **5al**.
(Schema 1, **5ma**): Analog 5aa anstelle 1-Octen wird 3-Cyclohexyl-1-propen = **1m** (232 µl, 1,5 mmol) verwendet und ergibt das Produkt **5ma.**
(Schema 1, **5ka**): Analog 5aa anstelle 1-Octen wird 6-Chlor-1-hexen = **1k** (198.5 µl, 1.5 mmol) verwendet und ergibt das Produkt **5ka**.
(Schema 1, **5ia**): Analog 5aa anstelle 1-Octen wird 7-Methyl-3-methylocta-1,6-dien = 1i (272,9 µl, 1,5 mmol) verwendet und ergibt das Produkt **5ia**.

## Patentansprüche

1. Verfahren zur Herstellung von α,β-ungesättigten Aldehyden aus Alkenen, **gekennzeichnet durch** Umsetzung eines Alkens (Olefins) in Gegenwart eines Synthesegases aus Kohlenmonoxid und Wasserstoff sowie eines Rhodiumkatalysators in Kombination mit einem organischen Phosphorliganden und einem Cokatalysator aus einem organischen Amin und einer schwachen organischen Säure in einem organischen Lösungsmittel,
wobei als Alkene terminale Alkene, Cycloalkene und aromatische Olefine mit einer Kohlenstoffzahl von 2 bis 40 oder Gemische eingesetzt werden, die gegebenenfalls substituiert sind.

2. Verfahren nach Anspruch 1, **dadurch gekennzeichnet, dass** ein Synthesegas mit einem Verhältnis CO:H₂ im Bereich von 10:1 bis 1:10 eingesetzt wird.

3. Verfahren nach Anspruch 1 oder 2, **dadurch gekennzeichnet, dass** das organische Lösungsmittel ausgewählt ist aus der Gruppe umfassend Essigsäure(methyl, ethyl oder n-butyl)ester und N-Methylpyrrolidon.

4. Verfahren nach einem der Ansprüche 1 bis 3, **dadurch gekennzeichnet, dass** als organisches Amin, ein primäres oder sekundäres Amineingesetzt wird, wobei die Verbindungen gegebenenfalls substituiert sind.

5. Verfahren nach einem der Ansprüche 1 bis 4, **dadurch gekennzeichnet, dass** die schwache organische Säure ausgewählt ist aus der Gruppe umfassend C₆-C₁₅-aromatische Carbonsäuren, aliphatische C₁-C₂₀-Carbonsäuren und heteroaromatische Carbonsäuren, wobei die Verbindungen gegebenenfalls substituiert sind.

6. Verfahren nach einem der Ansprüche 1 bis 5, **dadurch gekennzeichnet, dass** als Cokatalysator eine Kombination aus Pyrrolidin und Benzoesäure eingesetzt wird.

7. Verfahren nach einem der Ansprüche 1 bis 6, **dadurch gekennzeichnet, dass** die Reaktionstemperatur bei 20 bis 150°C liegt.

8. Verfahren nach einem der Ansprüche 1 bis 7, **dadurch gekennzeichnet, dass** der Rhodiumkatalysator ein Rhodiumkomplex ist, der mindestens eine der folgenden Verbindungen enthält: CO und/oder Olefin, Halogenid, Tetrafluoroborat, Hydrid, Carboxylat und/oder Sulfat.

9. Verfahren nach einem der Ansprüche 1 bis 8, **dadurch gekennzeichnet, dass** die Phosphorliganden monodentate Phosphine, Phosphite, Phosphonite, Phosphinite oder bidentate Phosphine, Phosphonite, Phospite, Phosphinite bzw. gemischte bidentate Liganden wie Phosphin/Phosphitkombinationen sind, bei denen der Phosphor an Aryl-und/oder (Cyclo)alkyl-, Aryloxy- und/oder (Cyclo)alkoxygruppen gebunden ist.

10. Verfahren nach einem der Ansprüche 1 bis 9, **dadurch gekennzeichnet, dass** das Verhältnis von Rhodium : Ligand bei der Verwendung monodentater Liganden 1: 50 beträgt.

11. Verfahren nach einem der Ansprüche 1 bis 10, **dadurch gekennzeichnet, dass** der Druck im Bereich von 1 bis 50 bar liegt.

12. Verwendung des Verfahrens nach einem der Ansprüche 1 bis 11 zur Herstellung von Kreuzaldolkondensaten ungesättigter Aldehyde, wobei neben einem Olefin ein aromatisches oder heteroaromatisches Aldehyd als Ausgangsstoff und kein Lösungsmittel oder ein polares Lösungsmittel eingesetzt wird.

13. Verfahren nach einem der Ansprüche 1 bis 11, **dadurch gekennzeichnet, dass** als Lösungsmittel Dimethylformamid oder N-Methyl-2-pyrollidon eingesetzt werden.

## Claims

1. Process for preparing α,β-unsaturated aldehydes from alkenes, **characterized by** conversion of an alkene (olefin) in the presence of a synthesis gas composed of carbon monoxide and hydrogen and of a rhodium catalyst in combination with an organic phosphorus ligand and a cocatalyst composed of an organic amine and a weak organic acid in an organic solvent,
wherein the alkenes used are terminal alkenes, cycloalkenes and aromatic olefins having a carbon number of 2 to 40 or mixtures thereof, which are optionally substituted.

2. Process according to Claim 1, **characterized in that** a synthesis gas having a CO:H₂ ratio of 10:1 to 1:10 is used.

3. Process according to Claim 1 or 2, **characterized in that** the organic solvent is selected from the group comprising methyl acetate, ethyl acetate, n-butyl acetate and N-methylpyrrolidone.

4. Process according to any of Claims 1 to 3, **characterized in that** the organic amine used is a primary or secondary amine, where the compounds are optionally substituted.

5. Process according to any of Claims 1 to 4, **characterized in that** the weak organic acid is selected from the group comprising C₆-C₁₅ aromatic carboxylic acids, aliphatic C₁-C₂₀ carboxylic acids and heteroaromatic carboxylic acids, where the compounds are optionally substituted.

6. Process according to any of Claims 1 to 5, **characterized in that** the cocatalyst used is a combination of pyrrolidine and benzoic acid.

7. Process according to any of Claims 1 to 6, **characterized in that** the reaction temperature is 20 to 150°C.

8. Process according to any of Claims 1 to 7, **characterized in that** the rhodium catalyst is a rhodium complex containing at least one of the following compounds: CO and/or olefin, halide, tetrafluoroborate, hydride, carboxylate and/or sulphate.

9. Process according to any of Claims 1 to 8, **characterized in that** the phosphorus ligands are monodentate phosphines, phosphites, phosphonites, phosphinites or bidentate phosphines, phosphonites, phosphites, phosphinites or mixed bidentate ligands such as phosphine/phosphite combinations in which the phosphorus is bonded to aryl and/or (cyclo)alkyl, aryloxy and/or (cyclo)alkoxy groups.

10. Process according to any of Claims 1 to 9, **characterized in that** the ratio of rhodium:ligand in the case of use of monodentate ligands is 1:50.

11. Process according to any of Claims 1 to 10, **characterized in that** the pressure is in the range from 1 to 50 bar.

12. Use of the process according to any of Claims 1 to 11 for preparation of cross-aldol condensates of unsaturated aldehydes, wherein, as well as an olefin, an aromatic or heteroaromatic aldehyde as starting material and no solvent or a polar solvent is used.

13. Process according to any of Claims 1 to 11, **characterized in that** the solvent used is dimethylformamide or N-methyl-2-pyrrolidone.

## Revendications

1. Procédé de fabrication d'aldéhydes α,β-insaturés à partir d'alcènes, **caractérisé par** la mise en réaction d'un alcène (oléfine) en présence d'un gaz de synthèse constitué de monoxyde de carbone et d'hydrogène, ainsi que d'un catalyseur de rhodium en combinaison avec un ligand phosphore organique et un co-catalyseur constitué d'une amine organique et d'un acide organique faible dans un solvant organique,
en tant qu'alcènes, des alcènes terminaux, des cycloalcènes et des oléfines aromatiques ayant un nombre de carbones de 2 à 40 ou des mélanges étant utilisés, qui sont éventuellement substitués.

2. Procédé selon la revendication 1, **caractérisé en ce qu'**un gaz de synthèse ayant un rapport CO: H₂ dans la plage allant de 10:1 à 1:10 est utilisé.

3. Procédé selon la revendication 1 ou 2, **caractérisé en ce que** le solvant organique est choisi dans le groupe comprenant l'ester méthylique, éthylique ou n-butylique de l'acide acétique et la N-méthylpyrrolidone.

4. Procédé selon l'une quelconque des revendications 1 à 3, **caractérisé en ce qu'**une amine primaire ou secondaire est utilisée en tant qu'amine organique, les composés étant éventuellement substitués.

5. Procédé selon l'une quelconque des revendications 1 à 4, **caractérisé en ce que** l'acide organique faible est choisi dans le groupe comprenant les acides carboxyliques aromatiques en C₆-C₁₅, les acides carboxyliques aliphatiques en C₁-C₂₀ et les acides carboxyliques hétéroaromatiques, les composés étant éventuellement substitués.

6. Procédé selon l'une quelconque des revendications 1 à 5, **caractérisé en ce qu'**une combinaison de pyrrolidine et d'acide benzoïque est utilisée en tant que co-catalyseur.

7. Procédé selon l'une quelconque des revendications 1 à 6, **caractérisé en ce que** la température de réaction est de 20 à 150 °C.

8. Procédé selon l'une quelconque des revendications 1 à 7, **caractérisé en ce que** le catalyseur de rhodium est un complexe de rhodium, qui contient au moins un des composés suivants : CO et/ou une oléfine, un halogénure, un tétrafluoroborate, un hydrure, un carboxylate et/ou un sulfate.

9. Procédé selon l'une quelconque des revendications 1 à 8, **caractérisé en ce que** les ligands phosphore sont des phosphines, phosphites, phosphonites, phosphinites monodentates ou des phosphines, phosphonites, phosphites, phosphinites bidentates ou des ligands bidentates mixtes, tels que des combinaisons phosphine/phosphite, dans lesquels le phosphore est relié à des groupes aryle et/ou (cyclo)alkyle, aryloxy et/ou (cyclo)alcoxy.

10. Procédé selon l'une quelconque des revendications 1 à 9, **caractérisé en ce que** le rapport rhodium:ligand lors de l'utilisation de ligands monodentates est de 1:50.

11. Procédé selon l'une quelconque des revendications 1 à 10, **caractérisé en ce que** la pression se situe dans la plage allant de 1 à 50 bar.

12. Utilisation du procédé selon l'une quelconque des revendications 1 à 11 pour la fabrication de condensats aldoliques croisés d'aldéhydes insaturés, un aldéhyde aromatique ou hétéroaromatique étant utilisé en plus d'une oléfine en tant que matière première et aucun solvant ou un solvant polaire étant utilisé.

13. Procédé selon l'une quelconque des revendications 1 à 11, **caractérisé en ce que** le diméthylformamide ou la N-méthyl-2-pyrrolidone est utilisé en tant que solvant.
